Europäisches Patentamt

European Patent Office

Office européen des brevets

(11) Publication number: **0 351 876**
**A2**

(12) # EUROPEAN PATENT APPLICATION

(21) Application number: 89113454.6

(22) Date of filing: 21.07.89

(51) Int. Cl.⁴: **A61K 37/02 , A61K 39/00 , //C07K13/00**

(30) Priority: 22.07.88 JP 183083/88

(43) Date of publication of application:
24.01.90 Bulletin 90/04

(84) Designated Contracting States:
**DE FR GB IT**

(71) Applicant: AJINOMOTO CO., INC.
5-8, Kyobashi 1-chome, Chuo-ku
Tokyo 104(JP)

Applicant: Kishimoto, Tadamitsu, Prof.
3-5-31, Nakano
Tondabayashi Osaka-fu(JP)

(72) Inventor: Kishimoto, Tadamitsu
No. 3-5-31, Nakano
Tondabayashi-shi Osaka-fu(JP)
Inventor: Hirano, Toshio
No. 19, Mihigaoka
Ibaraki-shi Osaka-fu(JP)
Inventor: Akiyama, Yukio Central research
Lab. Ajinomoto Co.
Inc. No. 1-1, Suzuki-cho
Kawasaki-ku Kawasaki-shi
Kanagawa-ken(JP)
Inventor: Okano, Akira Central research Lab.
Ajinomoto Co.
Inc. No. 1-1, Suzuki-cho
Kawasaki-ku Kawasaki-shi
Kanagawa-ken(JP)

(74) Representative: Strehl, Schübel-Hopf,
Groening
Maximilianstrasse 54 Postfach 22 14 55
D-8000 München 22(DE)

(54) Composition for potentiating vaccination effect.

(57) A composition which cmprises human B cell differentiation factor ("human BCDF") as an effective ingredient may be used for potentiating the vaccination effect of vaccines.

## COMPOSITION FOR POTENTIATING VACCINATION EFFECT

Field of the Invention

The present invention relates to a composition for potentiating the vaccination effect which is useful for potentiating the antibody producing effect of a vaccine used for immunizing humans or animals against infection with pathogenic microorganisms such as bacteria, viruses, etc.

More particularly, the present invention relates to compositions for potentiating the vaccination effect comprising human B cell differentiation factor (BCDF) as the effective ingredient.

Prior art

Mature B cells which are activated by stimulation with antigen proliferate with the assistance of T cells. It is known that B cells differentiate into antibody-producing cells by the action of a monocyte-derived differentiation-inducing substance. The presence of this substance was elucidated by R.W. Dutton et al. (Transplant. Ref., 23, 66 (1975)) and A. Schimpl and E. Wecher et al. (Nature N. Biol., 237, 15 (1972)).

Since then, functional proof suggesting the presence of such factors in mice and humans has been accumulated. These factors are collectively called B cell differentiation factor (BCDF).

It was also proposed to term human BCDF as BSF-2 or interleukin 6 (IL-6) (Nature, 324, 73 (1986), EMBO. J., 6, 1219 (1987)). In this specification, however, the conventional term "BCDF" is used. Furthermore, human BCDF has no interferon activity and is thus different from IFN-$\beta_2$ standard having an interferon activity (European Patent Application Laid Open No. 0220574).

Human BCDF possesses activity which is important in the human body. Extensive research has resulted in the determination of the DNA sequence and amino acid sequence of hBCDF (Japanese Patent Application Laid-Open Nos. 42688/88 and 56291/88) and in a process for the production of human BCDF using E. coli (Japanese Patent Application No. 302699/86). It has been found that human BCDF can be an immunotherapeutic agent effective for treatment of infectious diseases and cancers (Japanese Patent Application Nos 116332/87 and 289007/87).

Compositions for potentiating the vaccination effect which comprise human BCDF and conventional vaccines are briefly described below.

The importance of using a vaccine for preventing viral infections has become known, for example, from the fact that smallpox was extinguished on the earth by vaccination. Live vaccine has been developed against viruses of nubeola, rubella, mumps, etc. It is considered that by spreading vaccination with live vaccine, a crusade against these diseases could be successful. Furthermore, inactivated vaccine or subunit vaccine has been increasingly used as a prophylactic agent against rabies, influenza and Japanese encephalitis. In general, for the production of vaccine, it is necessary to proliferate viruses in cell culture.

Many viruses, however, such as hepatitis B virus, are difficult to culture. Furthermore, in the case of viruses which cause a continuous infection over long time periods, it is considered that attenuated live vaccine would be dangerous. Therefore recently two different types of new vaccines have been developed, i. e. a subunit vaccine and a peptide vaccine. The subunit vaccine comprises among the antigens on the viral surface only the portion which is important for the prevention of invention, which subunit is produced in E. coli, yeast etc. in large quantities. The peptide vaccine is produced by synthesizing a certain peptide based on the information on the amino acid sequence on the antigens on the viral surface. These new types of vaccines are advantageous, since they can be produced in large quantities even if the virus itself may not be cultured under usual conditions or if safety restrictions prohibit the culture of a dangerous virus. However, subunit vaccine and synthetic peptide vaccine involve the problem that the produced antigen protein is soluble and its immunogenicity is poor, that is, the antigen protein has merely a poor ability to induce immune response in the host. In order to make such a vaccine practically usable, it is necessary to increase the immunogenicity and it is thus necessary to use an immunoadjuvant (immunopotentia tor). In the course of developing such immunoadjuvants, however, only aluminum gel adjuvants such as aluminum hydroxide gel, aluminum phosphate gel, potash alum, etc. are currently used in the vaccination of humans. When these aluminum gels are administered in a high dose, there is the danger of accelerted IgE production resulting in allergic conditions in humans. Recently, adjuvant activator MDP (N-acetylmuramyl-L-alanyl-D-isoglutamine) occurring in cell walls and its derivatives have been considered as a promising adjuvant. However, if they are administered in a high dose, side effects such as pyrexia, etc. are reported because the adjuvant has a structure derived from bacteria. Hence there are problems in the practical use of these compounds. In order to make subunit vaccine or synthetic peptide vaccine practically usable, it is necessary to develop an effective adjuvant having minimized side effects or a vaccination effect potentiator capable of potentiating the effect of existing adjuvants and allowing to reduce the dose of adjuvant or the dose of antigen.

Hitherto such desirable compositions for potentiating the vaccination response which have minimized side effects and which show a controllable effect have not been reported.

When applying vaccines that have been currently used in practice, there are low responder groups in which immune response to vaccine is weak and effective immunization is not achieved. For example, in the group of patients with the age of 50 years or more the percentage of Hbs antibody-negative patients reached 25 % even after subcutaneous administration of hepatitis B vaccine 3 times (Results of hepatitis B vaccination test (Phase III) (Liver, Gallbladder & Pancreas), 9 (3), 463-479 (1984)). As a countermeasure against such low respondse, it has been attempted to increase the frequency of administration. However, any agent for potentiating the vaccination which can directly act on host cells responsible for immunity is yet unknown.

Summary of the Invention

Accordingly, an object of the present invention is to provide an effective composition for potentiating the vaccination effect which can potentiate immune response to vaccine and can reduce the amounts of adjuvant and antigen necessary for achieving immunity.

As a result of extensive investigations to solve the foregoing problem, the present inventors have found that compositions for potentiating the vaccination effect comprising human BCDF as the effective ingredient can accelerate the immune response to vaccine including the production of antibodies in the living body and have thus accomplished the present invention.

The present invention relates to compositions for potentiating the vaccination effect comprising human BCDF as the effective ingredient.

The human BCDF in accordance with the present invention has the following amino acid sequence (I) or (II) shown, for example, in Japanese Patent Application Laid-Open Nos. 42688/88 and 289007/87.

Amino acid sequence (I):

Pro Val Pro Pro Gly Glu Asp Ser Lys Asp Val
Ala Ala Pro His Arg Gln Pro Leu Thr Ser Ser
Glu Arg Ile Asp Lys Gln Ile Arg Tyr Ile Leu
Asp Gly Ile Ser Ala Leu Arg Lys Glu Thr Cys
Asn Lys Ser Asn Met Cys Glu Ser Ser Lys Glu
Ala Leu Ala Glu Asn Asn Leu Asn Leu Pro Lys
Met Ala Glu Lys Asp Gly Cys Phe Gln Ser Gly
Phe Asn Glu Glu Thr Cys Leu Val Lys Ile Ile
Thr Gly Leu Leu Glu Phe Glu Val Tyr Leu Glu
Tyr Leu Gln Asn Arg Phe Glu Ser Ser Glu Glu
Gln Ala Arg Ala Val Gln Met Ser Thr Lys Val
Leu Ile Gln Phe Leu Gln Lys Lys Ala Lys Asn
Leu Asp Ala Ile Thr Thr Pro Asp Pro Thr Thr
Asn Ala Ser Leu Leu Thr Lys Leu Gln Ala Gln
Asn Gln Trp Leu Gln Asp Met Thr Thr His Leu
Ile Leu Arg Ser Phe Lys Glu Phe Leu Gln Ser
Ser Leu Arg Ala Leu Arg Gln Met
or

Amino acid sequence (II):

Ala Pro Val Pro Pro Gly Glu Asp Ser Lys Asp
Val Ala Ala Pro His Arg Gln Pro Leu Thr Ser
Ser Glu Arg Ile Asp Lys Gln Ile Arg Tyr Ile
Leu Asp Gly Ile Ser Ala Leu Arg Lys Glu Thr

Cys Asn Lys Ser Asn Met Cys Glu Ser Ser Lys
Glu Ala Leu Ala Glu Asn Asn Leu Asn Leu Pro
Lys Met Ala Glu Lys Asp Gly Cys Phe Gln Ser
Gly Phe Asn Glu Glu Thr Cys Leu Val Lys Ile
Ile Thr Gly Leu Leu Glu Phe Glu Val Tyr Leu
Glu Tyr Leu Gln Asn Arg Phe Glu Ser Ser Glu
Glu Gln Ala Arg Ala Val Gln Met Ser Thr Lys
Val Leu Ile Gln Phe Leu Gln Lys Lys Ala Lys
Asn Leu Asp Ala Ile Thr Thr Pro Asp Pro Thr
Thr Asn Ala Ser Leu Leu Thr Lys Leu Gln Ala
Gln Asn Gln Trp Leu Gln Asp Met Thr Thr His
Leu Ile Leu Arg Ser Phe Lys Glu Phe Leu Gln
Ser Ser Leu Arg Ala Leu Arg Gln Met

Amino acid sequence (I) is the sequence of human BCDF (human BCDF having amino acid sequence (I) is hereinafter referred to as human BCDF) and amino acid sequence (II) is a polypeptide having a structure obtained by adding one Ala to human BCDF at the N-terminus thereof (hereinafter referred to as human Ala-BCDF). However, human BCDF used in the present invention is not necessarily restricted to the structure shown by the amino acid sequence (I) or (II) described above.

That is, biological equivalents of human BCDF, i.e., polypeptides having a structure formed by adding one or a plurality of amino acids to human BCDF at the N-terminus and/or C-terminus thereof and those having a structure in which one or a plurality of amino acids in the human BCDF structure are replaced by other amino acids can be used as the human BCDF of the present invention, so long as the human BCDF activity is maintained. Preferably, human BCDF or human Ala-BCDF is used. In the composition for potentiating the vaccination effect the content of the human BCDF in accordance with the present invention is 0.0001 to 100 wt%, preferably 0.1 to 1.0 wt%.

Furthermore, the vaccination effect-potentiating composition of the present invention comprising as the effective ingredient the human BCDF may contain usual stabilizers such as serum albumin etc., and a conventional excipient such as mannitol etc.

The vaccination effect potentiating agent of the present invention may be administered by any route, i. e. intravenously, intramuscularly or subcutaneously.

The vaccination effect-potentiating agent may also be administered simultaneously or after a vaccine is administered. In the case of administering the potentiating agent at the same time as a vaccine, it is convenient to mix the potentiating agent with the vaccine and/or adjuvant.

Compositions containing human BCDF and one or more cytokines such as interleukin 1 (IL-1), interleukin 2 (IL-2), interleukin 3 (IL-3), interleukin 4 (IL-4), interleukin 5 (IL-5), interferon γ (γ-IFN), granulocyte-macrophage colony stimulating factor (GM-CSF), macrophage colony stimulating factor (M-CSF) and granulocyte colony stimulating factor (G-CSF), in addition to BCDF, are also useful for potentiating the antibody producing affect of vaccine.

The human BCDF used in the present invention may be any of those produced and purified from human T cells, B cells, fibroblasts, etc. by known method (Proc. Natl. Acad. Sci., USA, 82, 5490 (1985)) and those produced by culturing transformants obtained by introducing a gene coding for human BCDF into an appropriate host such as E. coli, yeast, monkey cells (COS cells), hamster, bacteria, etc. using a suitable vector and further purifying the transformants.

With respect to details of these production methods, reference is made to Japanese Patent Application Laid-Open Nos. 115024/86 and 56291/88 and Japanese Patent Application No. 302699/86, and to European Patent Application 0 257 406.

Effects

The composition of the present invention comprising human BCDF as the effective ingredient principally can potentiate the production of antibodies to a vaccine, that is, can potentiate immune response of host so that the amount of antigen and the amount of adjuvent which are apt to cause side effects in the living body can be reduced. In addition, the composition of the present invention can improve poor response to a certain vaccine antigen and the reduced immune response observed in the case of aged people.

Hereafter the present invention is described in more detail by referring to the following examples.

4

Example 1

Medical preparation of human BCDF

The HPLC fraction of human BCDF or human Ala-BCDF was allowed to stand at -20 °C overnight and acetonitrile in the upper phase was removed. The lower phase was subjected to gel filtration with Sephadex G-25 or dialysis to remove the remaining acetonitrile and TFA and substitute with PBS solution. The solution was diluted and if necessary serum albumin (0.1%) or serum (2 to 10%) from human or optionally from another mammal was added to the diluted solution. Thereafter, the system is subjected to sterile filtration to give a human BCDF preparation or human Ala-BCDF preparation.

Example 2

Potentiation of the immune response to a soluble protein antigen by administration of human BCDF in vivo

An adjuvant, namely Freund complete adjuvant which causes potent immune response in DBA/2 mice in vivo, can however not be practically used since it contains tubercule bacillus or Freund incomplete adjuvant containing no tubercule bacillus but having a poor activity was emulsified together with 50 μg myoglobin/mouse and the emulsion was intraperitoneally administered. A suspension having a definite concentration of human BCDF in PBS containing 1% mouse serum was subcutaneously given to mice in a dose of 100 μl each for 6 consecutive days from the day of administration.

Seven days later serum was collected from the mice and the amount of myoglobin-specific antibody in blood was determined by ELISA. The antibody titer is expressed by reciprocal number log 2 of the dilution number which shows the same OD value. As shown in Table 1, the amount of anti-myoglobin antibody in blood induced by the weak adjuvant (Freund incomplete adjuvant) increased by 3 times compared to the group to which no human BCDF had been administered. Furthermore, the concentration of anti-myoglobin antibody in blood which was increased by human BCDF was comparable to the concentration of antibody in blood induced by strong adjuvant (Freund complete adjuvant).

### Table 1

| Adjuvant | Administration of Human BCDF | Antibody Titer* of Anti-Myoglobin | |
|---|---|---|---|
| FCA | – | $3.0 \pm 0.7$ | (4) |
| FCA | + | $3.3 \pm 0.5$ | (4) |
| FIA | – | $1.2 \pm 0.05$ | (4) |
| FIA | + | $3.2 \pm 0.05$ | (4) |

(a)

\* Numerical value within parenthesis denotes the number of animals used.

a: FIA was used; there was a significant difference in p of less than 5 %.

Example 3

Potentiation of aluminum gel adjuvant by administration of human BCDF in vivo.

Myoglobin was adsorbed on aluminum gel adjuvant which is the only adjuvant admitted by the authorities. 5 μg of myoglobin/mouse was intraperitoneally administered to mice. Administration and blood collection were performed according to the same schedule as in Example 2 and antigen-specific antibody titer in blood was determined. As shown in Table 2, by the administration of human BCDF the amount of anti-myoglobin antibody induced by aluminum gel adjuvant increased by 3 times as compared to the group to which no human BCDF had been administered.

## Table 2

| Administration of Human BCDF | Antibody Titer[*] of Anti-Myoglobin | |
|---|---|---|
| – | $1.0 \pm 0.08$ (5) | (a) |
| + | $2.9 \pm 0.43$ (5) | |

[*]   Numerical value in parenthesis denotes the number of animals used.

a:   There was a significant difference in p of less than 5%.

Example 4

Potentiation of immune response to hepatitis B vaccine by human BCDF administration.

Human plasma-derived hepatitis B antigen Hbs antigen was adsorbed to aluminum gel and 2 μg of Hbs/mouse was intraperitoneally administered to mice. Administration and blood collection were performed according to the same schedule as in Example 2 and antigen-specific antibody titer in blood was determined.

As shown in Table 3, the amount of Hbs-specific antibody increased by the administration of human Ala-BCDF.

Table 3

| Administration of Human Ala-BCDF | Antibody Titer[*] of Anti-Hepatitis B Antigen |
|---|---|
| − | $2.0 \pm 0.08$ (4) |
| + | $3.9 \pm 0.54$ (4) |

(a)

[*]  Numerical value in parenthesis denotes the number of animals used.

a:  There was a significant difference in p of less than 5%.

## Claims

1. The use of human B-cell differentiation factor, or its biological equivalent in a therapeutically acceptable amount for preparing a composition for potentiating the vaccination effect of a vaccine.

2. The use according to claim 1, wherein said human BCDF has the following amino acid sequence (I):

Pro Val Pro Pro Gly Glu Asp Ser Lys Asp Val
Ala Ala Pro His Arg Gln Pro Leu Thr Ser Ser
Glu Arg Ile Asp Lys Gln Ile Arg Tyr Ile Leu
Asp Gly Ile Ser Ala Leu Arg Lys Glu Thr Cys
Asn Lys Ser Asn Met Cys Glu Ser Ser Lys Glu
Ala Leu Ala Glu Asn Asn Leu Asn Leu Pro Lys
Met Ala Glu Lys Asp Gly Cys Phe Gln Ser Gly
Phe Asn Glu Glu Thr Cys Leu Val Lys Ile Ile
Thr Gly Leu Leu Glu Phe Glu Val Tyr Leu Glu
Tyr Leu Gln Asn Arg Phe Glu Ser Ser Glu Glu
Gln Ala Arg Ala Val Gln Met Ser Thr Lys Val
Leu Ile Gln Phe Leu Gln Lys Lys Ala Lys Asn
Leu Asp Ala Ile Thr Thr Pro Asp Pro Thr Thr
Asn Ala Ser Leu Leu Thr Lys Leu Gln Ala Gln
Asn Gln Trp Leu Gln Asp Met Thr Thr His Leu
Ile Leu Arg Ser Phe Lys Glu Phe Leu Gln Ser
Ser Leu Arg Ala Leu Arg Gln Met

3. The use according to claim 1, wherein said biological equivalent of human BCDF has the following amino acid sequence (II):

Ala Pro Val Pro Pro Gly Glu Asp Ser Lys Asp
Val Ala Ala Pro His Arg Gln Pro Leu Thr Ser
Ser Glu Arg Ile Asp Lys Gln Ile Arg Tyr Ile
Leu Asp Gly Ile Ser Ala Leu Arg Lys Glu Thr
Cys Asn Lys Ser Asn Met Cys Glu Ser Ser Lys
Glu Ala Leu Ala Glu Asn Asn Leu Asn Leu Pro
Lys Met Ala Glu Lys Asp Gly Cys Phe Gln Ser
Gly Phe Asn Glu Glu Thr Cys Leu Val Lys Ile
Ile Thr Gly Leu Leu Glu Phe Glu Val Tyr Leu

7

Glu Tyr Leu Gln Asn Arg Phe Glu Ser Ser Glu
Glu Gln Ala Arg Ala Val Gln Met Ser Thr Lys
Val Leu Ile Gln Phe Leu Gln Lys Lys Ala Lys
Asn Leu Asp Ala Ile Thr Thr Pro Asp Pro Thr
Thr Asn Ala Ser Leu Leu Thr Lys Leu Gln Ala
Gln Asn Gln Trp Leu Gln Asp Met Thr Thr His
Leu Ile Leu Arg Ser Phe Lys Glu Phe Leu Gln
Ser Ser Leu Arg Ala Leu Arg Gln Met

4. The use according to claim 1, wherein said human BCDF is produced by procaryotic cells.

5. The use according to any of the claims 1 to 4, wherein the composition comprises at least one member selected from the group consisting of interleukin 1 (IL-1), interleukin 2 (IL-2), interleukin 3 (IL-3), interleukin 4 (IL-4), interleukin 5 (IL-5), $\gamma$-interferon ($\gamma$-IFN), granulocyte macrophage colony stimulating factor (GM-CSF), macrophage colony stimulating factor (M-CSF), and granulocyte colony stimulating factor (G-CSF), in addition to BCDF.